# EUROPÄISCHE PATENTSCHRIFT

(11) **EP 0 472 131 B1**
(45) Veröffentlichungstag und Bekanntmachung des Hinweises auf die Patenterteilung: **30.10.1996**
(21) Anmeldenummer: 91113821.2
(22) Anmeldetag: 17.08.1991
(51) Int. Cl.: G01N 27/18, G01N 33/00

(54) **Verfahren zur Bestimmung der Konzentration einer Komponente eines aus mehreren Komponenten bestehenden Gasgemisches durch Messung der Wärmeleitfähigkeit**
Method for determining the concentration of a component in a gas mixture containing several components by measuring thermal conductivity
Procédé pour déterminer la concentration d'un composant d'un mélange gazeux contenant plusieurs composants en mesurant la conductivité thermique

(30) Priorität: 20.08.1990 DE 4026266
(43) Veröffentlichungstag der Anmeldung: 26.02.1992
(73) Patentinhaber: Hartmann & Braun Aktiengesellschaft, D-60484 Frankfurt am Main (DE)
(72) Erfinder: Hielscher, Bernd, Dr., W-6368 Bad Vilbel 3 (DE); Horn, Bertold, Dr., W-6382 Friedrichsdorf (DE); Liedtke, Thomas, W-6370 Oberursel (DE)
(74) Vertreter: Meissner, Peter E., Dipl.-Ing.

(56) Entgegenhaltungen:
- DD-A- 13 378
- DE-A- 2 710 394
- FR-A- 2 574 181
- PATENT ABSTRACTS OF JAPAN vol. 8, no. 97 (P-272)(1534) 08 Mai 1984, & JP-A-59 009 559
- WPI DATABASE Derwent Publications Ltd., LONDON GB accession no. 78-26910A, week 7814; & SU-A-543 862

## Beschreibung

Die Erfindung bezieht sich auf ein verfahren zur Bestimmung der Konzentration einer Komponente eines aus mehreren Komponenten bestehenden Gasgemisches durch Messung der Wärmeleitfähigkeit gemäß dem Oberbegriff der nebengeordneten Ansprüche 1 bis 3.

Aus der DE-PS 37 11 511 ist ein verfahren zur Bestimmung der Konzentration von Komponenten eines aus mehreren Komponenten bestehenden Gasgemisches durch Messung der Wärmeleitfähigkeit bekannt. Bei dem bekannten verfahren werden die Wärmeleitfähigkeitswerte des Gasgemisches bei verschiedenen Gastemperaturen gemessen. Die Anzahl der Gastemperaturen muß dabei mindestens so groß sein wie die um 1 verringerte Anzahl der Komponenten, aus denen das Gasgemisch besteht. Aus den gemessenen Wärmeleitfähigkeitswerten wird dann mit bekannten mathematischen Methoden zur Lösung nichtlinearer Gleichungssysteme die Konzentration der einzelnen Komponenten des Gasgemisches errechnet. Das bekannte verfahren ist in der praktischen Anwendung von der Auflösung her auf aus maximal drei Komponenten bestehende Gasgemische beschränkt. Andererseits gibt es Fälle, in denen nur die Konzentration einer speziellen Komponente eines aus mehr als zwei Komponenten bestehenden Gasgemisches bestimmt werden soll, wie z. B. bei der Bestimmung der Konzentration von Tetrachlorethen (PER) im Inneren von chemischen Reinigungsanlagen.

Ein verfahren zur Bestimmung der Konzentration einer Komponente eines aus mehreren Komponenten bestehenden Gasgemisches durch Messung der Wärmeleitfähigkeit gemäß dem Oberbegriff der nebengeordneten Ansprüche 1 bis 3 ist aus der SU-Patentschrift 543 862 bekannt. Das Gasgemisch, das die Komponente enthält, deren Konzentration bestimmt werden soll, wird einem ersten Wärmeleitfähigkeitssensor zugeführt. Das den ersten Wärmeleitfähigkeitssensor verlassende Gasgemisch wird einer Einrichtung zugeführt, die diejenige Komponente, deren Konzentration bestimmt werden soll, aus dem Gasgemisch extrahiert. Danach wird das Gasgemisch ohne die Komponente, deren Konzentration bestimmt werden soll, einem zweiten Wärmeleitfähigkeitssensor zugeführt. Die beiden Wärmeleitfähigkeitssensoren bilden zwei Brückenwiderstände einer elektrischen Brückenschaltung, deren Ausgangsspannung als Maß für die zu bestimmende Konzentration der extrahierten Gaskomponente ist. Mit dem aus der SU-PS 543 862 bekannten Verfahren ist es nicht möglich, die Konzentration von sich schnell ändernden Gaszusammensetzungen zu bestimmen. Die dem zweiten Wärmeleitfähigkeitssensor vorgeschaltete Einrichtung, die diejenige Komponente des Gasgemisches, deren Konzentration bestimmt werden soll, aus dem Gasgemisch extrahiert, verursacht eine Verlängerung der Laufzeit des dem zweiten Wärmeleitfähigkeitssensor zugeführten Gasgemisches. Die Gasgemische, die den beiden Wärmeleitfähigkeitssensoren jeweils zu demselben Zeitpunkt zugeführt werden, entsprechen daher Konzentrationen der Gasgemische zu unterschiedlichen Zeitpunkten. Die Messung ist daher mit einem systematischen Fehler behaftet.

Der Erfindung liegt die Aufgabe zugrunde, ein Verfahren der eingangs genannten Art anzugeben, das es erlaubt, den zeitlichen Verlauf der Konzentration einer sich schnell ändernden Komponente eines aus mehr als zwei Komponenten bestehenden Gasgemisches zu bestimmen.

Diese Aufgabe wird erfindungsgemäß durch die in dem kennzeichnenden Teil der nebengeordneten Patentansprüche 1, 2 bzw. 3 angegebenen Merkmale gelöst. Die erfindungsgemäßen Verfahren erlauben eine praktisch kontinuierliche Messung des zeitlichen Verlaufs der Konzentration einer einzelnen

Komponente eines aus mehreren Komponenten bestehenden Gasgemisches, und zwar unabhängig von Änderungen der Konzentration der anderen Komponenten des Gasgemisches. Nach den erfindungsgemäßen Verfahren arbeitende Vorrichtungen lassen sich kostengünstig und in kompakter Bauweise herstellen.

Vorteilhafte Weiterbildungen des Verfahrens nach den Patentansprüchen 1, 2 oder 3 sind in den Patentansprüchen 4 bis 6 gekennzeichnet.

Die erfindungsgemäßen Verfahren werden im folgenden anhand von in den Zeichnungen dargestellten Vorrichtungen zur Durchführung der Verfahren näher erläutert. Als Ausführungsbeispiel sind drei verschiedene Vorrichtungen zur Bestimmung der Konzentration von Tetrachlorethen (PER) im Inneren von chemischen Reinigungsanlagen gewählt worden. Bei dem Gas im Inneren von chemischen Reinigungsanlagen handelt es sich um ein Gasgemisch, das z. B. aus Luft (Stickstoff, Sauerstoff, Argon, Kohlendioxid sowie Wasser) und PER als Komponenten besteht. Die Komponente des Gasgemisches, deren Konzentration bestimmt werden soll, ist PER. Die Extraktion des PER aus dem Gasgemisch erfolgt in den Ausführungsbeispielen durch Adsorption, z. B. mit Adsorbentien für die Gaschromatographie. Es zeigen
- Figur 1: das Blockschaltbild einer Vorrichtung zur Bestimmung der Konzentration von PER gemäß der ersten Lösungsvariante,
- Figur 2: das Blockschaltbild einer Vorrichtung zur Bestimmung der Konzentration von PER gemäß der zweiten Lösungsvariante und
- Figur 3: das Blockschaltbild einer Vorrichtung zur Bestimmung der Konzentration von PER gemäß der dritten Lösungsvariante.
Gleiche Bauteile sind mit den gleichen Bezugszeichen versehen.

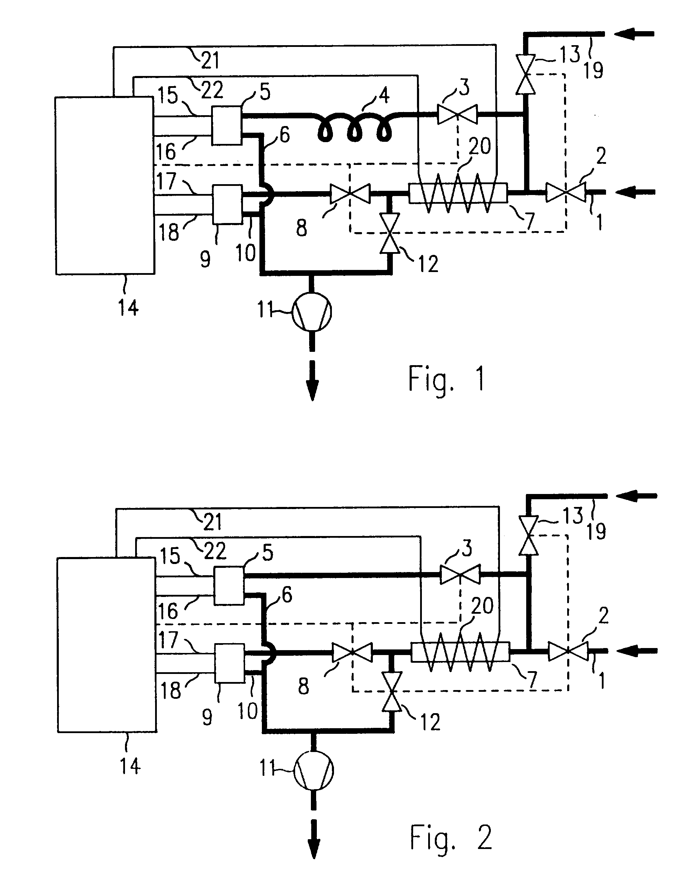

Die Figur 1 zeigt das Blockschaltbild einer ersten Vorrichtung zur Bestimmung der Konzentration von PER im Inneren von chemischen Reinigungsanlagen. Das aus dem Inneren einer chemischen Reinigungsanlage entnommene Gasgemisch ist im folgenden als Meßgas bezeichnet. Das Meßgas wird der in der Figur 1 dargestellten Vorrichtung über eine Gasleitung 1 zugeführt. Hinter einem ersten Ventil 2 teilt sich der Meßgasstrom in zwei Teilströme. Der erste Teilstrom des Meßgases fließt über ein weiteres Ventil 3 sowie einen Schlauch 4 in einen ersten Wärmeleitfähigkeitssensor 5. Der Schlauch 4 dient als Einrichtung zur Verlängerung der Laufzeit des ersten Teilstromes des Meßgases. Das Meßgas verläßt den Wärmeleitfähigkeitssensor 5 über eine Gasleitung 6. Der zweite Teilstrom des Meßgases fließt über einen Adsorber 7 und ein weiteres Ventil 8 zu einem zweiten Wärmeleitfähigkeitssensor 9. Der Adsorber 7 adsorbiert das in dem zweiten Teilstrom des Meßgases enthaltene PER. Das dem zweiten Wärmeleitfähigkeitssensor 9 zugeführte Gasgemisch verläßt den Wärmeleitfähigkeitssensor 9 über eine Gasleitung 10. Eine Pumpe 11 ist mit den Gasleitungen 6 und 10 verbunden. Sie saugt das Meßgas durch den Wärmeleitfähigkeitssensor 5 sowie das den Adsorber 7 verlassende Gasgemisch durch den Wärmeleitfähigkeitssensor 9 und führt diese Gasgemische in das Innere der chemischen Reinigungsanlage zurück. In der Figur 1 sind zusätzlich zu den Ventilen 2, 3 und 8 zwei weitere Ventile 12 und 13 dargestellt. Eine Elektronik-Schaltung 14 steuert über gestrichelt dargestellte Signalleitungen die Ventile 2, 3 und 8 sowie 12 und 13 nach einem vorgegebenen Programm so, daß während des eigentlichen Meßvorganges die Ventile 2, 3 und 8 geöffnet und die Ventile 12 und 13 geschlossen sind. Der Wärmeleitfähigkeitssensor 5 liefert über elektrische Leitungen 15 und 16 ein elektrisches Signal, das ein Maß für die Wärmeleitfähigkeit des über den Wärmeleitfähigkeitssensor 5 fließenden Gasgemisches ist, an die Elektronik-Schaltung 14. In gleicher Weise liefert der Wärmeleitfähigkeitssensor 9 über elektrische Leitungen 17 und 18 ein elektrisches Signal, das ein Maß für die Wärmeleitfähigkeit des über den Wärmeleitfähigkeitssensor 9 fließenden Gasgemisches ist, an die Elektronik-Schaltung 14. Um eine Temperaturdifferenz zwischen den Wärmeleitfähigkeitssensoren 5 und 9 zu vermeiden, ist es vorteilhaft, sie auf einem gemeinsamen Block zu montieren. Die Elektronik-Schaltung 14 bildet die Differenz zwischen den von den Wärmeleitfähigkeitssensoren 5 und 9 gemessenen Leitfähigkeitswerten als Maß für die Konzentration des in dem Meßgas enthaltenen PER.

Der Schlauch 4 ist so dimensioniert, daß sein Innenquerschnitt und seine Länge an das zeitliche Verhalten des Gasdurchtritts durch den Adsorber 7 so angepaßt sind, daß den beiden Wärmeleitfähigkeitssensoren 5 und 9 Gasgemische zugeführt werden, die bis auf die nachzuweisende Komponente in ihrem zeitlichen Verlauf identisch sind.

Die Elektronik-Schaltung 14 kann eine Anzeigeeinrichtung enthalten, die das Ergebnis direkt anzeigt. Das Ergebnis kann aber auch, z. B. in Form eines eingeprägten Stromes, an eine externe Anzeigeeinrichtung weitergeleitet werden. Überschreitet die Konzentration des PER einen ersten vorgebbaren Grenzwert, können Alarmeinrichtungen betätigt werden. Andererseits kann die Verriegelungseinrichtung der Beschickungsöffnung einer chemischen Reinigungsanlage durch die Elektronik-Schaltung so gesteuert werden, daß erst dann ein Öffnen möglich ist, wenn ein zweiter vorgebbarer Grenzwert für die Konzentration des PER unterschritten worden ist.

Das bisher beschriebene Verfahren ist durch die Kapazität des Adsorptionsmittels in der praktischen Anwendung - abhängig von dem Volumen des Adsorbers - auf einige Stunden begrenzt. Ist das Adsorptionsmittel mit PER gesättigt, wird das Adsorptionsmittel mit einem Spülgas, das keine von dem Adsorptionsmittel adsorbierbare Komponente enthält, wie z. B. Luft, gespült, bis es regeneriert ist. Das Spülgas wird der Vorrichtung über eine Gasleitung 19 zugeführt. Während des Regeneriervorganges, dessen Beginn und Dauer von der Elektronik-Schaltung 14 gesteuert wird, sind die Ventile 2, 3 und 8 geschlossen, die Ventile 12 und 13 sind in dieser Zeit geöffnet. Die Pumpe 11 saugt jetzt das der Gasleitung 19 zugeführte Spülgas über den Adsorber 7 und leitet es in das Innere der chemischen Reinigungsanlage. Das PER wird dabei aus dem Adsorptionsmittel entfernt. Dieser Vorgang läßt sich durch Erhöhen der Temperatur des Adsorptionsmittels beschleunigen. Eine Heizwicklung 20, die über elektrische Leitungen 21 und 22 mit der Elektronik-Schaltung 14 verbunden ist, erhitzt das in dem Adsorber 7 enthaltene Adsorptionsmittel während des Regeneriervorganges. Durch die Temperaturerhöhung vermindert sich das Adsorptionsvermögen des Adsorptionsmittels. Erhöht man die Temperatur des Adsorptionsmittels auf Werte, bei denen fast keine Adsorption mehr erfolgt, kann die Regenerierung des Adsorptionsmittels auch mit einem PER-haltigen Gasgemisch, wie z. B. dem Meßgas, erfolgen.

Die Figur 2 zeigt das Blockschaltbild einer zweiten Vorrichtung zur Bestimmung der Konzentration von PER im Inneren von chemischen Reinigungsanlagen. Dieses Blockschaltbild ist bis auf den Schlauch 4 mit dem in der Figur 1 dargestellten Blockschaltbild identisch. Der erste Teilstrom des Meßgases ist über das Ventil 3 dem ersten Wärmeleitfähigkeitssensor 5 direkt zugeführt. Um die durch den Adsorber 7 verursachte Laufzeitverzögerung des zweiten Teilstromes des Meßgases auszugleichen, ist in der Elektronik-Schaltung 14 eine elektronische Einrichtung vorgesehen, die das Ausgangssignal des ersten Wärmeleitfähigkeitssensors 5 entsprechend der Verweildauer des zweiten Teilstromes des Meßgases in dem Adsorber 7 verzögert. Derartige Einrichtungen zur Verzögerung von elektrischen Signalen sind an sich bekannt und daher in der Figur 2 nicht dargestellt. In diesem Ausführungsbeispiel dient die Differenz zwischen dem verzögerten Ausgangssignal des ersten Wärmeleitfähigkeitssensors 5 und dem Ausgangssignal des zweiten Wärmeleitfähigkeitssensors 9 als Maß für die Konzentration der Komponente des Gasgemisches, deren Konzentration bestimmt werden soll.

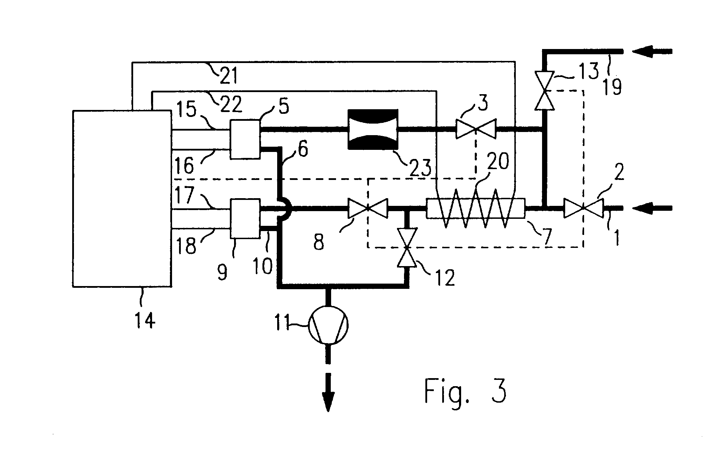

Die Figur 3 zeigt das Blockschaltbild einer dritten Vorrichtung zu Bestimmung der Konzentration von PER im Inneren von chemischen Reinigungsanlagen. Dieses Blockschaltbild ist bis auf den Schlauch 4 mit dem in der Figur 1 dargestellten Blockschaltbild identisch. Anstelle des Schlauches 4 ist eine Drossel 23 vorgesehen. Diese Drossel verringert den über den ersten Wärmeleitfähigkeitssensor 5 fließenden Teilstrom des Meßgases derart gegenüber dem über den Adsorber 7 fließenden Teilstrom des Meßgases, daß für jede der beiden Gasstrecken zwischen der Verzweigungsstelle des Gasgemisches (Ventil 2) und dem ersten bzw. dem zweiten Wärmeleitfähigkeitssensor 5 bzw. 9 der Quotient aus dem Volumen der jeweiligen Gasstrecke und dem zugehörigen Teilstrom gleich ist.

In allen drei Ausführungsbeispielen ist es möglich, unterschiedliche Meßeigenschaften der beiden Wärmeleitfähigkeitssensoren 5 und 9 durch zusätzliche Maßnahmen zu kompensieren. Hierzu wird während eines Korrekturvorganges den Wärmeleitfähigkeitssensoren 5 und 9 gleichzeitig dasselbe Gas zugeführt. Die hierfür erforderlichen zusätzlichen Leitungen und Ventile sind aus Gründen der Übersichtlichkeit in den Figuren 1 und 2 nicht dargestellt. Weichen während des Korrekturvorganges die elektrischen Ausgangssignale der beiden Wärmeleitfähigkeitssensoren 5 und 9 voneinander ab, speichert die Elektronik-Schaltung 14 diesen Wert und berücksichtigt ihn bis zum nächsten Korrekturvorgang als Korrekturwert bei der Bildung der Differenz zwischen den von den Wärmeleitfähigkeitssensoren 5 und 9 gemessenen Wärmeleitfähigkeitswerten.

Der in der DE-PS 37 11 511 beschriebene Wärmeleitfähigkeitssensor ist wegen seiner geringen Größe und des damit verbundenen kleinen Gasvolumens besonders gut für die Durchführung der erfindungsgemäßen Verfahren geeignet.

## Patentansprüche

1. Verfahren zur Bestimmung der Konzentration einer Komponente eines aus mehreren Komponenten bestehenden Gasgemisches durch Messung der Wärmeleitfähigkeit,
- bei dem das Gasgemisch mit der Komponente, deren Konzentration bestimmt werden soll, einem ersten Wärmeleitfähigkeitssensor zugeführt wird, der die Wärmeleitfähigkeit des Gasgemisches mißt,
- bei dem diejenige Komponente, deren Konzentration bestimmt werden soll, aus dem Gasgemisch extrahiert und das Gasgemisch ohne die Komponente, deren Konzentration bestimmt werden soll, einem zweiten Wärmeleitfähigkeitssensor zugeführt wird, der die Wärmeleitfähigkeit des Gasgemisches ohne die Komponente, deren Konzentration bestimmt werden soll, mißt und
- bei dem die Differenz der beiden Wärmeleitfähigkeitswerte als Maß für die Konzentration der Komponente des Gasgemisches, deren Konzentration bestimmt werden soll, gebildet wird,
dadurch gekennzeichnet,
- daß das Gasgemisch mit der Komponente, deren Konzentration bestimmt werden soll, in zwei Teilströme aufgeteilt wird,
- daß der erste Teilstrom des Gasgemisches dem ersten Wärmeleitfähigkeitssensor über eine Einrichtung zur Verlängerung der Laufzeit dieses Teilstromes des Gasgemisches zugeführt wird,
- daß der zweite Teilstrom des Gasgemisches der Einrichtung zugeführt wird, die aus ihm die Komponente extrahiert, deren Konzentration bestimmt werden soll, und danach der Teilstrom des Gasgemisches ohne die Komponente, deren Konzentration bestimmt werden soll, dem zweiten Wärmeleitfähigkeitssensor zugeführt wird, und
- daß die Dauer der Verlängerung der Laufzeit des ersten Teilstromes des Gasgemisches entsprechend der Verweildauer des zweiten Teilstromes des Gasgemisches in der Einrichtung, die die Komponente extrahiert, deren Konzentration bestimmt werden soll, gewählt wird.

2. Verfahren zur Bestimmung der Konzentration einer Komponente eines aus mehreren Komponenten bestehenden Gasgemisches durch Messung der Wärmeleitfähigkeit,
- bei dem das Gasgemisch mit der Komponente, deren Konzentration bestimmt werden soll, einem ersten Wärmeleitfähigkeitssensor zugeführt wird, der die Wärmeleitfähigkeit des Gasgemisches mißt,
- bei dem diejenige Komponente, deren Konzentration bestimmt werden soll, aus dem Gasgemisch extrahiert und das Gasgemisch ohne die Komponente, deren Konzentration bestimmt werden soll, einem zweiten Wärmeleitfähigkeitssensor zugeführt wird, der die Wärmeleitfähigkeit des Gasgemisches ohne die Komponente, deren Konzentration bestimmt werden soll, mißt und
- bei dem die Differenz der beiden Wärmeleitfähigkeitswerte als Maß für die Konzentration der Komponente des Gasgemisches, deren Konzentration bestimmt werden soll, gebildet wird,
dadurch gekennzeichnet,
- daß das Gasgemisch mit der Komponente, deren Konzentration bestimmt werden soll, in zwei Teilströme aufgeteilt wird,
- daß der erste Teilstrom des Gasgemisches dem ersten Wärmeleitfähigkeitssensor zugeführt wird,
- daß der zweite Teilstrom des Gasgemisches der Einrichtung zugeführt wird, die aus ihm die Komponente extrahiert, deren Konzentration bestimmt werden soll, und danach der Teilstrom des Gasgemisches ohne die Komponente, deren Konzentration bestimmt werden soll, dem zweiten Wärmeleitfähigkeitssensor zugeführt wird und
- daß das Ausgangssignal des ersten Wärmeleitfähigkeitssensors gegenüber demjenigen des zweiten Wärmeleitfähigkeitssensors entsprechend der Verweildauer des zweiten Teilstromes des Gasgemisches in der Einrichtung, die die Komponente extrahiert, deren Konzentration bestimmt werden soll, verzögert wird, wobei die Differenz zwischen dem verzögerten Ausgangssignal des ersten Wärmeleitfähigkeitssensors und dem Ausgangssignal des zweiten Wärmeleitfähigkeitssensors als Maß für die Konzentration der Komponente des Gasgemisches, deren Konzentration bestimmt werden soll, dient.

3. Verfahren zur Bestimmung der Konzentration einer Komponente eines aus mehreren Komponenten bestehenden Gasgemisches durch Messung der Wärmeleitfähigkeit,
- bei dem das Gasgemisch mit der Komponente, deren Konzentration bestimmt werden soll, einem ersten Wärmeleitfähigkeitssensor zugeführt wird, der die Wärmeleitfähigkeit des Gasgemisches mißt,
- bei dem diejenige Komponente, deren Konzentration bestimmt werden soll, aus dem Gasgemisch extrahiert und das Gasgemisch ohne die Komponente, deren Konzentration bestimmt werden soll, einem zweiten Wärmeleitfähigkeitssensor zugeführt wird, der die Wärmeleitfähigkeit des Gasgemisches ohne die Komponente, deren Konzentration bestimmt werden soll, mißt und
- bei dem die Differenz der beiden Wärmeleitfähigkeitswerte als Maß für die Konzentration der Komponente des Gasgemisches, deren Konzentration bestimmt werden soll, gebildet wird,
dadurch gekennzeichnet,
- daß das Gasgemisch mit der Komponente, deren Konzentration bestimmt werden soll, in zwei Teilströme aufgeteilt wird,
- daß der erste Teilstrom des Gasgemisches dem ersten Wärmeleitfähigkeitssensor zugeführt wird,
- daß der zweite Teilstrom des Gasgemisches der Einrichtung zugeführt wird, die aus ihm die Komponente extrahiert, deren Konzentration bestimmt werden soll, und danach der Teilstrom des Gasgemisches ohne die Komponente, deren Konzentration bestimmt werden soll, dem zweiten Wärmeleitfähigkeitssensor zugeführt wird und
- daß die Aufteilung des Gasgemisches in zwei Teilströme so erfolgt, daß für jede der beiden Gasstrecken zwischen der Verzweigungsstelle des Gasgemisches und dem ersten bzw. dem zweiten Wärmeleitfähigkeitssensor der Quotient aus dem Volumen der jeweiligen Gasstrecke und dem zugehörigen Teilstrom gleich ist.

4. Verfahren nach einem der Ansprüche 1 bis 3, dadurch gekennzeichnet, daß die Extraktion der Komponente, deren Konzentration bestimmt werden soll, durch ein Adsorptionsmittel erfolgt.

5. Verfahren nach Anspruch 4, dadurch gekennzeichnet, daß die Regenerierung des Adsorptionsmittels durch Spülen des Adsorptionsmittels mit einem Gas erfolgt, das keine von dem Adsorptionsmittel adsorbierbare Komponente enthält.

6. Verfahren nach Anspruch 4, dadurch gekennzeichnet, daß die Regenerierung des Adsorptionsmittels durch Erhöhen der Temperatur des Adsorptionsmittels auf Werte erfolgt, bei denen sich das Adsorptionsvermögen des Adsorptionsmittels stark vermindert.

## Claims

1. A method for determining the concentration of a constituent of a gas mixture consisting of a plurality of constituents, by measuring the thermal conductivity,
- in which the gas mixture with the constituent, the concentration of which is to be determined, is fed to a first thermal conductivity sensor which measures the thermal conductivity of the gas mixture,
- in which the constituent, the concentration of which is to be determined, is extracted from the gas mixture and the gas mixture without the constituent, the concentration of which is to be determined, is fed to a second thermal conductivity sensor, which measures the thermal conductivity of the gas mixture without the constituent, the concentration of which is to be measured, and
- in which the difference between the two thermal conductivity values is formed as a measurement of the concentration of the constituent of the gas mixture, the concentration of which is to be measured,
characterised in that
- the gas mixture with the constituent, the concentration of which is to be determined, is divided up into two partial streams,
- that the first partial stream of the gas mixture is fed to the first thermal conductivity sensor via a means for prolonging the transit time of this partial stream of the gas mixture,
- that the second partial stream of the gas mixture is fed to the means which extracts therefrom the constituent, the concentration of which is to be determined, and thereafter the partial stream of the gas mixture, without the constituent, the concentration of which is to be determined, is fed to the second thermal conductivity sensor, and
- that the duration of the prolongation of the transit time of the first partial stream of the gas mixture is selected corresponding to the dwell time of the second partial stream of the gas mixture in the means which extracts the constituent, the concentration of which is to be determined.

2. A method for determining the concentration of a constituent of a gas mixture consisting of a plurality of constituents, by measuring the thermal conductivity,
- in which the gas mixture with the constituent, the concentration of which is to be determined, is fed to a first thermal conductivity sensor which measures the thermal conductivity of the gas mixture,
- in which the constituent, the concentration of which is to be determined, is extracted from the gas mixture and the gas mixture without the constituent, the concentration of which is to be determined, is fed to a second thermal conductivity sensor, which measures the thermal conductivity of the gas mixture without the constituent, the concentration of which is to be measured, and
- in which the difference between the two thermal conductivity values is formed as a measurement of the concentration of the constituent of the gas mixture, the concentration of which is to be measured,
characterised in that
- the gas mixture with the constituent, the concentration of which is to be determined, is divided up into two partial streams,
- that the first partial stream of the gas mixture is fed to the first thermal conductivity sensor,
- that the second partial stream of the gas mixture is fed to the means which extracts therefrom the constituent, the concentration of which is to be determined, and thereafter the partial stream of the gas mixture, without the constituent, the concentration of which is to be determined, is fed to the second thermal conductivity sensor, and
- that the output signal of the first thermal conductivity sensor is delayed relative to that of the second thermal conductivity sensor corresponding to the dwell time of the second partial stream of the gas mixture in the means which extracts the constituent, the concentration of which is to be determined, the difference between the delayed output signal of the first thermal conductivity sensor and the output signal of the second thermal conductivity sensor serving as a measurement of the concentration of the constituent of the gas mixture, the concentration of which is to be determined.

3. A method for determining the concentration of a constituent of a gas mixture consisting of a plurality of constituents, by measuring the thermal conductivity,
- in which the gas mixture with the constituent, the concentration of which is to be determined, is fed to a first thermal conductivity sensor which measures the thermal conductivity of the gas mixture,
- in which the constituent, the concentration of which is to be determined, is extracted from the gas mixture and the gas mixture without the constituent, the concentration of which is to be determined, is fed to a second thermal conductivity sensor, which measures the thermal conductivity of the gas mixture without the constituent, the concentration of which is to be measured, and
- in which the difference between the two thermal conductivity values is formed as a measurement of the concentration of the constituent of the gas mixture, the concentration of which is to be measured,
characterised in that
- the gas mixture with the constituent, the concentration of which is to be determined, is divided up into two partial streams,
- that the first partial stream of the gas mixture is fed to the first thermal conductivity sensor,
- that the second partial stream of the gas mixture is fed to the means which extracts therefrom the constituent, the concentration of which is to be determined, and thereafter the partial stream of the gas mixture, without the constituent, the concentration of which is to be determined, is fed to the second thermal conductivity sensor, and
- that the division of the gas mixture into two partial streams is effected such that for each of the two gas paths between the branching point of the gas mixture and the first or the second thermal conductivity sensor the quotient of the volume of the respective gas path and the associated partial stream is identical.

4. A method according to one of Claims 1 to 3, characterised in that the extraction of the constituent, the concentration of which is to be determined, is effected by an adsorption agent.

5. A method according to Claim 4, characterised in that the regeneration of the adsorption agent is effected by rinsing the adsorption agent with a gas which contains no constituent adsorbable by the adsorption agent.

6. A method according to Claim 4, characterised in that the regeneration of the adsorption agent is effected by raising the temperature of the adsorption agent to values at which the adsorption ability of the adsorption agent is greatly reduced.

## Revendications

1. Procédé pour déterminer la concentration de composants d'un mélange de gaz constitué de plusieurs composants, par la mesure de la conductibilité thermique,
- dans lequel le mélange de gaz avec les composants, dont la concentration doit être déterminée, est amené à un premier capteur de conductibilité thermique, qui mesure la conductibilité thermique du mélange de gaz,
- dans lequel les composants, dont la concentration doit être déterminée, sont extraits du mélange de gaz, et le mélange de gaz sans les composants, dont la concentration doit être déterminée, est amené à un second capteur de conductibilité thermique, qui mesure la conductibilité thermique du mélange de gaz sans les composants, dont la concentration doit être déterminée, et
- dans lequel la différence des deux valeurs de conductibilité thermique est formée comme mesure pour la concentration des composants du mélange de gaz, dont la concentration doit être déterminée,
caractérisé,
- en ce que le mélange de gaz avec les composants, dont la concentration doit être déterminée, est réparti en deux courants partiels,
- en ce que le premier courant partiel du mélange de gaz est amené au premier capteur de conductibilité thermique, par l'intermédiaire d'un dispositif pour prolonger le temps de parcours de ce courant partiel du mélange de gaz,
- en ce que le second courant partiel du mélange de gaz est amené au dispositif qui en extrait les composants, dont la concentration doit être déterminée, et ensuite le courant partiel du mélange de gaz sans les composants, dont la concentration doit être déterminée, est amené au second capteur de conductibilité thermique, et
- en ce que la durée du prolongement du temps de parcours du premier courant partiel du mélange de gaz est choisie en correspondance à la durée de séjour du second courant partiel du mélange de gaz dans le dispositif qui extrait les composants, dont la concentration doit être déterminée.

2. Procédé pour déterminer la concentration de composants d'un mélange de gaz constitué de plusieurs composants, par la mesure de la conductibilité thermique,
- dans lequel le mélange de gaz avec les composants, dont la concentration doit être déterminée, est amené à un premier capteur de conductibilité thermique, qui mesure la conductibilité thermique du mélange de gaz,
- dans lequel les composants, dont la concentration doit être déterminée, sont extraits du mélange de gaz, et le mélange de gaz sans les composants, dont la concentration doit être déterminée, est amené à un second capteur de conductibilité thermique, qui mesure la conductibilité thermique du mélange de gaz sans les composants, dont la concentration doit être déterminée, et
- dans lequel la différence des deux valeurs de conductibilité thermique est formée comme mesure pour la concentration des composants du mélange de gaz, dont la concentration doit être déterminée,
caractérisé,
- en ce que le mélange de gaz avec les composants, dont la concentration doit être déterminée, est réparti en deux courants partiels,
- en ce que le premier courant partiel du mélange de gaz est amené au premier capteur de conductibilité thermique,
- en ce que le second courant partiel du mélange de gaz est amené au dispositif qui en extrait les composants, dont la concentration doit être déterminée, et ensuite le courant partiel du mélange de gaz sans les composants, dont la concentration doit être déterminée, est amené au second capteur de conductibilité thermique, et
- en ce que le signal de sortie du premier capteur de conductibilité thermique est retardé par rapport à celui du second capteur de conductibilité thermique, en correspondance à la durée de séjour du second courant partiel du mélange de gaz dans le dispositif qui extrait les composants, dont la concentration doit être déterminée, la différence entre le signal de sortie retardé du premier capteur de conductibilité thermique et le signal de sortie du second capteur de conductibilité thermique servant de mesure pour la concentration des composants du mélange de gaz, dont la concentration doit être déterminée.

3. Procédé pour déterminer la concentration de composants d'un mélange de gaz constitué de plusieurs composants, par la mesure de la conductibilité thermique,
- dans lequel le mélange de gaz avec les composants, dont la concentration doit être déterminée, est amené à un premier capteur de conductibilité thermique, qui mesure la conductibilité thermique du mélange de gaz,
- dans lequel les composants, dont la concentration doit être déterminée, sont extraits du mélange de gaz, et le mélange de gaz sans les composants, dont la concentration doit être déterminée, est amené à un second capteur de conductibilité thermique, qui mesure la conductibilité thermique du mélange de gaz sans les composants, dont la concentration doit être déterminée, et
- dans lequel la différence des deux valeurs de conductibilité thermique est formée comme mesure pour la concentration des composants du mélange de gaz, dont la concentration doit être déterminée,
caractérisé,
- en ce que le mélange de gaz avec les composants, dont la concentration doit être déterminée, est réparti en deux courants partiels,
- en ce que le premier courant partiel du mélange de gaz est amené au premier capteur de conductibilité thermique,
- en ce que le second courant partiel du mélange de gaz est amené au dispositif qui en extrait les composants, dont la concentration doit être déterminée, et ensuite le courant partiel du mélange de gaz sans les composants, dont la concentration doit être déterminée, est amené au second capteur de conductibilité thermique, et
- en ce que la répartition du mélange de gaz en deux courants partiels est réalisée de sorte que, pour chacun des deux parcours de gaz entre la zone de dérivation du mélange de gaz et respectivement les premier et second capteurs de conductibilité thermique, le quotient des volumes du parcours de gaz correspondant et du courant partiel associé est égal.

4. Procédé selon l'une des revendications 1 à 3,
caractérisé en ce que l'extraction des composants, dont la concentration doit être déterminée, est réalisée par un moyen d'adsorption.

5. Procédé selon la revendication 4,
caractérisé en ce que la régénération du moyen d'adsorption est réalisée par le rinçage du moyen d'adsorption par un gaz qui ne comporte aucun des composants adsorbés par le moyen d'adsorption.

6. Procédé selon la revendication 4,
caractérisé en ce que la régénération du moyen d'adsorption est réalisée par l'augmentation de la température du moyen d'adsorption à des valeurs, pour lesquelles le pouvoir d'adsorption du moyen d'adsorption est fortement réduit.
